# EUROPEAN PATENT APPLICATION

(11) **EP 4 548 913 A1**
(43) Date of publication of application: **07.05.2025**
(21) Application number: 23386111.1
(22) Date of filing: 06.11.2023
(51) Int. Cl.: A61K 9/20, A61K 31/155, A61K 31/522, A61P 3/10

(54) **A MONOLAYER TABLET OF LINAGLIPTIN AND METFORMIN**

(71) Applicant: Genepharm S.A., 15351 Pallini (GR)
(72) Inventor: XENOGIORGIS, Vasileios, 17676 Kallithea, Athens (GR); MPENEKIS, Vasilis, 11475 Athens (GR); MEGALOOIKONOMOU, Kalliopi, 15343 Ag. Paraskevi, Athens (GR)
(74) Representative: Roukounas, Dimitrios

(57) **Abstract**

A monolayer tablet comprising metformin granules and linagliptin granules, wherein the linagliptin granules are coated with a basic butylated methacrylate copolymer and the process of its preparation.

## Description

### FIELD OF THE INVENTION

The present invention relates to a monolayer tablet comprising linagliptin and metformin hydrochloride.

### BACKGROUND OF THE INVENTION

Linagliptin is a dipeptidyl peptidase IV (DPP-IV) inhibitor with the chemical name 1-[(4-methyl-quinazolin-2-yl)-methyl]-3-methyl-7-(2-butyn-l-yl)-8-(3-(R)-aminopi peridin-1-yl)-xanthine. Linagliptin is marketed as Tradjenta^{®}. Linagliptin is also marketed in combination with metformin hydrochloride. Metformin has the chemical name N, N-dimethyl biguanide and belongs to the biguanide class of compounds. The combination of linagliptin with metformin has the brand name Jentadueto^{®} and it is indicated in adults with type 2 diabetes mellitus. Tablets of Jentadueto^{®} are available in different strengths of linagliptin and metformin hydrochloride such as 2.5mg/500 mg, 2.5mg/850 mg and 2.5mg/1000 mg.

Compositions containing DPP-IV inhibitors are challenging as DPP-IV inhibitors with a primary or secondary amino group show incompatibilities with several standard excipients. The amino group appears to react with reducing sugars and with other reactive carbonyl groups.

Though the DPP-IV inhibitors themselves are very stable, they may react with incompatible antidiabetic compounds or their impurity products while preparing fixed combinations. The DPP-IV inhibitors may also react with many excipients used in solid dosage forms and with impurities of excipients, especially when in tight contact provided in tablets and at high excipient/drug ratios.

These unforeseen difficulties are primarily observed in low dosage ranges of the DPP-4 inhibitor used, in view of its high potency, and/or high dosage ranges of the incompatible antidiabetic compounds used. Thus, formulation development of a combination of linagliptin requires formulators which solve these technical problems, which may be associated with the low content of linagliptin in the formulation.

European Patent application 2285410 discloses pharmaceutical composition comprising linagliptin, metformin and one or more pharmaceutical excipients, and a nucleophilic and/or basic agents for stabilizing said DPP-IV inhibitor, linagliptin, against degradation. EP'410 exemplifies the use of a basic amino acid, L-arginine, to overcome the incompatibility issues and poor stability, which may be caused by reaction of linagliptin when combined with an incompatible metformin hydrochloride, or its impurity product and/or a pharmaceutical excipient having incompatible functional groups. Using a suitable nucleophilic and/or basic agent (e.g., a buffering and/or pH modifying agent) in pharmaceutical compositions helps in protecting the decomposition and degradation of linagliptin.

PCT application WO2015/107536 discloses combination of linagliptin and metformin HCl without any basic amino acid or stabilizer. '536 application exemplifies monolayer tablets of linagliptin and metformin hydrochloride using copovidone leading however to higher amounts of total impurities of linagliptin (Examples 1 and 2).

European application 3456319 discloses combination of linagliptin and metformin HCl in intimate admixture with a stearate.

The inventors of the present invention surprisingly found a chemically stable tablet composition comprising combination of linagliptin and metformin HCl that overcomes the above-mentioned problems.

### SUMMARY OF THE INVENTION

The present invention provides a monolayer tablet comprising
(a) metformin granules comprising metformin hydrochloride and one or more excipients;
(b) linagliptin granules comprising linagliptin or a pharmaceutically acceptable salt thereof and one or more excipients, wherein the linagliptin granules are coated with a basic butylated methacrylate copolymer; and optionally
(c) one or more extragranular excipients..

The present invention further provides a process for the preparation of a monolayer tablet comprising
(a) granulating metformin hydrochloride and one or more excipients to prepare metformin granules;
(b) granulating linagliptin or a pharmaceutically acceptable salt thereof and one or more excipients to prepare linagliptin granules and coating the linagliptin granules with a basic butylated methacrylate copolymer;
(c) mixing metformin granules prepared in (a) and linagliptin granules prepared in (b);
(d) optionally adding one or more extragranular excipients; and
(e) compressing into tablets.

### DETAILED DESCRIPTION OF THE INVENTION

The present invention provides a monolayer tablet of linagliptin and metformin hydrochloride.

Specifically, the present invention provides a monolayer tablet of linagliptin and metformin hydrochloride which does not have to comprise stabilizers of the active ingredients.

More specifically, the present invention provides a monolayer tablet of Linagliptin and metformin hydrochloride which does not have to comprise a basic amino acid, a stabilizer of the active ingredients and copovidone.

Thus, the present invention provides a monolayer tablet of linagliptin and metformin comprising (a) metformin granules comprising metformin hydrochloride and one or more excipients; (b) linagliptin granules comprising linagliptin or a pharmaceutically acceptable salt thereof and one or more excipients, wherein the linagliptin granules are coated with a basic butylated methacrylate copolymer; and optionally (c) one or more extragranular excipients.

The monolayer tablets may be film coated.

Linagliptin may be used in crystalline or amorphous form. Preferably, the tablet of the present invention comprises linagliptin form A or linagliptin form B or mixture of form A and form B. Linagliptin form A and linagliptin form B are disclosed in European patent application No. EP2016079 A1.

The D90 by volume of linagliptin, or of a pharmaceutically acceptable salt thereof, which is used in the preparation of the tablet of the present invention is preferably less than 200 µm (microns), more preferably, less than 50 microns as measured by laser light diffraction as described in European Pharmacopoeia (EP) 11th Edition 2.9.31.

Metformin hydrochloride may be used in crystalline or amorphous form. The D90 by volume of metformin hydrochloride which is used in the preparation of the tablet of the present invention is preferably less than 400 microns as measured by laser light diffraction as described in EP 11^{th} Edition 2.9.31.

The term "D90 by volume" means 90% of the volume of the particles have a diameter which is less than the specified diameter.

The weight ratio of metformin hydrochloride to linagliptin, or to a pharmaceutically acceptable salt thereof in the tablet, is preferably from 100:1 to 500:1.

Specific embodiments of a monolayer tablet of linagliptin and metformin hydrochloride of the present invention may be as follows -
(1) 2.5 mg of linagliptin and 500 mg metformin hydrochloride;
(2) 2.5 mg of linagliptin and 850 mg metformin hydrochloride;
(3) 2.5 mg of linagliptin and 1000 mg metformin hydrochloride.

The monolayer tablet of linagliptin and metformin hydrochloride of the present invention may be administered once or twice daily to the patient, preferably twice daily.

Pharmaceutical excipients which may be used in the monolayer tablet of the present invention, as intragranular and/or extragranular excipients, may be selected from the group consisting of one or more fillers or diluents, one or more binders, one or more lubricants, one or more disintegrants, one or more glidants, one or more sweeteners, one or more anti-adherents, one or more plasticizers, one or more colouring agents, one or more flavouring agents and one or more surfactants.

Suitable fillers may be selected, for example, from starch derivatives, such as corn starch, potato starch or rice starch; polysaccharides such as dextrins, maltodextrins, dextrates, microcrystalline cellulose, powdered cellulose, mixtures of microcrystalline cellulose and guar gum, co-processed blends of microcrystalline cellulose; and polyhydric alcohols, such as xylitol and sorbitol.

Suitable diluents may be selected, for example, from calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin and maltitol.

Suitable binders may be selected, for example, from acacia, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropyl methyl cellulose, maltodextrin, methylcellulose, polydextrose, polyethylene oxide, povidone, sodium alginate, starch paste, pregelatinized starch, sucrose, tragacanth, low-substituted hydroxypropyl cellulose, glucose and sorbitol.

Suitable disintegrants may be selected, for example, from alginic acid, carbon dioxide, carboxymethylcellulose calcium, carboxymethylcellulose sodium, microcrystalline cellulose, powdered cellulose, croscarmellose sodium, crospovidone, sodium docusate, guar gum, hydroxypropyl cellulose, methylcellulose, polacrilin potassium, poloxamer, povidone, sodium alginate, sodium glycine carbonate, sodium starch glycolate, starch, pregelatinized starch and low-substituted hydroxypropyl cellulose.

Suitable glidants may be selected, for example, from calcium silicate, powdered cellulose, starch, talc, colloidal silicon dioxide and magnesium trisilicate.

Suitable lubricants may be selected, for example, from magnesium stearate, stearic acid, sodium stearyl fumarate, sodium lauryl sulphate, magnesium lauryl sulphate, talc, polyethylene glycol and glyceryl behenate.

Suitable sweeteners may be selected, for example, from sugars, such as sucrose and glucose; cyclamate and salts thereof; saccharin and salts thereof; and aspartame.

Suitable flavouring agents may be selected, for example, from natural or synthetic flavours such as strawberry flavour, wild cherry flavour, green apple flavour, spearmint flavour, and peppermint flavour.

Suitable colouring agents may be selected, for example, from dyes such as: FD&C red #4, FD&C yellow #5; pigments such as: iron oxides (red, yellow, etc), Titanium dioxide, calcium carbonate; Lakes such as indigo, Aluminium Lakes and the like.

Suitable surfactants may be selected, for example, from sodium lauryl sulphate, copolymers of polyethylene oxide) and polypropylene oxide) (commercially called Poloxamers or Poloxamines), Polysorbates such as Tween^{®} 20, Tween^{®} 80 and the like.

Suitable stabilizers may be selected, for example, from antioxidants, ascorbic acid, citric acid, phosphoric acid, BHT, BHA and the like.

Suitable plasticizers may be selected, for example, from polysorbate 80, polyethylene glycol, propylene glycol, hydroxypropyl cellulose and the like.

Suitable anti-adherents may be selected, fir example, from talc, polyethylene glycol, hydrogenated castor oil, glyceryl behenate and the like.

Preferably, the metformin granules of the tablet of the present invention comprise as intragranular excipients a filler and a binder. More preferably, the metformin granules comprise as intragranular excipients microcrystalline cellulose and hydroxypropyl cellulose.

Preferably, the linagliptin granules of the tablet of the present invention comprise as intragranular excipients a filler, a disintegrant and a binder. More preferably, the linagliptin granules comprise as intragranular excipients maize starch, mannitol, pregelatinized starch and povidone.

The linagliptin granules in the tablet of the present invention comprising linagliptin or a pharmaceutically acceptable salt thereof and one or more excipients, are coated with a basic butylated methacrylate copolymer. Basic butylated methacrylate copolymer is a copolymer of 2-(dimethylamino)ethyl methacrylate, butyl methacrylate and methyl methacrylate. The ratio of 2-(dimethylamino)ethyl methacrylate groups to butyl methacrylate and methyl methacrylate groups is preferably about 2:1:1. Examples of basic butylated methacrylate copolymers include the commercially available Eurdagit^{®} products. Eudragit^{®} is the brand name for a diverse range of commercially available basic butylated methacrylate copolymers. Eudragit^{®} EPO, Eudragit^{®} E 100, Eudragit^{®} E 12.5, Eudragit^{®} RD 100 are examples of basic butylated methacrylate copolymers which may be used in the present invention. The coating of the linagliptin granules is used as a physical barrier between linagliptin and metformin.

According to a preferred embodiment, the monolayer tablet of the present invention is free of a basic amino acid, a stabilizer of the active ingredients and copovidone.

The present invention further provides a process for the preparation of the monolayer tablet of linagliptin and metformin.

The process for the preparation of a monolayer tablet comprises
(a) granulating metformin hydrochloride and one or more excipients to prepare metformin granules;
(b) granulating linagliptin or a pharmaceutically acceptable salt thereof and one or more excipients to prepare linagliptin granules and coating the linagliptin granules with basic butylated methacrylate copolymer;
(c) mixing metformin granules prepared in (a) and linagliptin granules prepared in (b);
(d) optionally adding one or more extragranular excipients; and
(e) compressing into tablets.

In the embodiments in which the tablet is coated, step (e) is followed by the step of coating the tablet.

The metformin granules may be prepared by processes well known in the art, such as wet or dry granulation. The linagliptin granules may be prepared by processes well known in the art, such as wet or dry granulation. Preferably, the metformin granules are wet granulated and the linagliptin granules are dry granulated.

The monolayer tablet of the present invention exhibits fast dissolution of both active ingredients. Thus, the dissolution of both metformin and linagliptin from the tablet of the present invention is preferably at least 50%, when the tablet is subjected to dissolution tests at pH range from 1.2 to 6.8 at 37° C ± 0.5°C using a USPII paddle apparatus with stirring speed of 50 rpm. More preferably, the dissolution of both metformin and linagliptin from the tablet of the present invention is at least 80%, when the tablet is subjected to dissolution tests at pH range from 1.2to 6.8 at 37° C ± 0.5°C using a USPII paddle apparatus with stirring speed of 50 rpm. Even more preferably, the dissolution of both metformin and linagliptin from the tablet of the present invention is at least 90%, when the tablet is subjected to dissolution tests at pH range from 1.2to 6.8 at 37° C ± 0.5°C using a USPII paddle apparatus with stirring speed of 50 rpm. Furthermore, the tablet of the present invention exhibits excellent stability of both active ingredients.

### EXAMPLES

### Comparative Example 1 - Reference listed Drug (RLD)

The table below shows the qualitative composition of the commercially available Jentadueto^{®} tablets.

| **Linagliptin/ Metformin** | |
|---|---|
| **Components** | **mg/tab** |
| Metformin HCl | 1000.0 |
| L-Arginine | NA |
| Linagliptin | 2.5 |
| p. Water* | q.s. |
| Aerosil 200 | NA |
| Maize Starch | NA |
| *Pre-lubrication Weight* | NA |
| Magnesium stearate | NA |
| *Core Tablet Weight* | NA |
| Opadry^{®} Pink | NA |
| ***Coated Tablet Weight*** | **~1200** |

| | |
|---|---|
| * Purified water is used in the granulation process (wet granulation) and it is not present in the tablet | |

### EXAMPLE 2

The present example shows a tablet according to the present invention and its process of manufacture.

| **Linagliptin/ Metformin** | **2.5/1000 mg** | |
|---|---|---|
| **Components** | **mg/tab** | **%** |
| **Wet Granulation 1** | | |
| Metformin HCl | 1000.0 | 78.7 |
| MCC 101 | 100.0 | 7.9 |
| Klucel^{®} HPC | 35.5 | 2.8 |
| *p. Water** | *q.s.* | q.s. |

| **Wet Granulation 2** | | |
|---|---|---|
| Linaqliptin | 2.5 | 0.2 |
| Maize Starch | 8.5 | 0.7 |
| Mannitol | 61.0 | 4.8 |
| Pregelatinized Starch | 8.5 | 0.7 |
| Povidone K30 | 2.5 | 0.2 |
| *p. Water** | *q.s*. | *q.s*. |

| **Spraying dispersion (only for Linagliptin granules)** | | |
|---|---|---|
| Eudragit EPO | 9.7 | 0.8 |
| SLS | 1.0 | 0.1 |
| Stearic Acid | 1.5 | 0.1 |
| Talc | 4.9 | 0.4 |

| Extragranular Excipients | | |
|---|---|---|
| Crospovidone CL-F | 18.0 | 1.4 |
| *Pre-lubrication Weight* | 1253.5 | 98.7 |
| Magnesium stearate | 14.0 | 1.3 |
| *Core Tablet Weight* | **1270.0** | **100.0** |
| Opadry^{®} Pink | 38.1 | 3.0 |
| ***Coated Tablet Weight*** | **1308.1** | **103.0** |

| | | |
|---|---|---|
| MCC: Microcrystalline cellulose HPC: Hydroxypropyl cellulose SLS: sodium lauryl sulphate * Purified water is used in the granulation process (wet granulation) and it is not present in the tablet | | |

**Step 1:** The Metformin API & MCC 101 are sieved (#30 mesh) and pre-mixed (Blend A)
**Step 2:** Klucel HPC is dissolved in appropriate qty of water
**Step 3:** Blend A is wet granulated with liquid of step 2
**Step 4:** Drying of the wet granules (Target LOD: 1.8-2.5%), sizing (#20 mesh) & mixing
**Step 5:** The intragranular excipients are sieved (#30 mesh) and sieved Linagliptin API is added (Blend B)
**Step 6:** Povidone K30 is dissolved in appropriate qty of water
**Step 7:** Blend B is wet granulated with liquid of step 6
**Step 8:** Drying of the wet granules (Target LOD: 1.5-3.0%), sizing & mixing
**Step 9:** Eudragit EPO mixture preparation
**Step 10:** Spraying of Dispersion of step 9 on Linagliptin granules (Target gain weight: 15-20%)
**Step 11:** Drying of the wet coated granules (Target LOD: 1.5-3.0%),sizing (#16 mesh) & mixing
**Step 12:** Mixing the sized granules of Metformin & Linagliptin (after corrections)
**Step 13:** Extra granular excipient (Crospovidone) is sieved and mixed (final mixing)
**Step 14:** Lubrication: Sieving of Magnesium Stearate (#60mesh) and mixing
**Step 15:** Compression
**Step 16:** Coating with Opadry^{®} Pink.

### COMPARATIVE EXAMPLES 3-6 with different binders:

### Comparative Example 3

The table below shows the composition of a tablet having the same excipients as the table of Example 2, including hydroxypropyl cellulose as binder, without the coating of the linagliptin granules.

| **Linagliptin/ Metformin** | **2.5/1000 mg** | |
|---|---|---|
| **Components** | **mg/tab** | **%** |
| Metformin HCl | 1000.0 | 80.65 |

| Intragranular for Metformin | | |
|---|---|---|
| MCC 101 | 100.0 | 8.06 |
| Hydroxypropyl cellulose | 37.5 | 3.02 |
| p. Water* | q.s. | q.s. |

| Intragranular for Linagliptin | | |
|---|---|---|
| Linagliptin | 2.5 | 0.2 |
| Maize starch | 8.5 | 0.69 |
| Mannitol 160C | 62.5 | 5.04 |
| Starch 1500 | 8.5 | 0.69 |
| Povidone K30 | 2.5 | 0.2 |
| p. Water* | q.s. | q.s. |
| *Pre-lubrication Weight* | *1222.0* | *98.55* |
| Magnesium stearate | 18.0 | 1.45 |
| *Core Tablet Weight* | **1240.0** | **100.0** |
| Opadry Pink | | 3.0 |
| ***Coated Tablet Weight*** | **1277.2** | **103.0** |

| | | |
|---|---|---|
| MCC: Microcrystalline cellulose * Purified water is used in the granulation process (wet granulation) and it is not present in the tablet | | |

**Step 1:** The Metformin API and MCC 101 are sieved and mixed.
**Step 2:** Hydroxypropyl cellulose is dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4
**Step 6:** (Presieved) Linagliptin is mixed with (presieved) Mannitol geometrically and then mixed with (presieved) maize starch and starch 1500.
**Step 7:** Povidone K30 is dissolved in water.
**Step 8:** Blend from step 6 is wet granulated with binding liquid from step 7
**Step 9:** Drying of the wet granules of step 8 (Target LOD: 1.5-3.0%)
**Step 10:** Sizing of the dried granules of step 9
**Step 11:** Sized granules of step 4 & 10 are mixed (unlubricated blend)
**Step 12:** Lubrication: Sieving of Magnesium Stearate (#60mesh) and mixing
**Step 13:** Compression
**Step 14:** Coating

### Comparative Example 4

The composition of the tablet shown in the table below, differs from the tablet of Example 1 in that it comprises Hydroxypropyl methylcellulose (HPMC) as binder and in that the linagliptin granules are not coated.

| **Linagliptin/ Metformin** | **2.5/1000 mg** | |
|---|---|---|
| **Components** | **mg/tab** | **%** |
| Metformin HCl | 1000.0 | 82.03 |

| Intragranular for Metformin | | |
|---|---|---|
| Maize starch | 30.0 | 2.46 |
| HPMC | 86.0 | 7.05 |
| p. Water* | q.s. | q.s. |

| Intragranular for Linagliptin | | |
|---|---|---|
| Linagliptin | 2.5 | 0.21 |
| Maize starch | 8.5 | 0.70 |
| Mannitol 160C | 62.5 | 5.13 |
| Starch 1500 | 8.5 | 0.70 |
| Povidone K30 | 2.5 | 0.21 |
| p. Water* | q.s. | q.s. |
| *Pre-lubrication Weight* | *1200.5* | *98.48* |
| Magnesium stearate | 18.5 | 1.52 |
| *Core Tablet Weight* | **1219.0** | **100.0** |
| Opadry Pink | | 3.0 |
| ***Coated Tablet Weight*** | **1255.6** | **103.0** |

| | | |
|---|---|---|
| * Purified water is used in the granulation process (wet granulation) and it is not present in the tablet | | |

**Step 1:** The Metformin API, Maize Starch and some qty of HPMC are sieved and mixed.
**Step 2:** Part of HPMC is dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4
**Step 6:** (Presieved) Linagliptin is mixed with (presieved) Mannitol geometrically and then mixed with (presieved) maize starch and starch 1500.
**Step 7:** Povidone K30 is dissolved in water.
**Step 8:** Blend from step 6 is wet granulated with binding liquid from step 7
**Step 9:** Drying of the wet granules of step 8 (Target LOD: 1.5-3.0%)
**Step 10:** Sizing of the dried granules of step 9
**Step 11:** Sized granules of step 4 & 10 are mixed geometrically (unlubricated blend)
**Step 12:** Lubrication: Sieving of Magnesium Stearate (#60mesh) and mixing
**Step 13:** Compression
**Step 14:** Coating

### Comparative Example 5

The composition of the tablet shown in the table below comprises Copovidone (Kollidon VA 64) as binder. The tablet does not contain coated linagliptin granules. Linagliptin is added to the binding liquid which is used in the wet granulation.

| **Linagliptin/ Metformin** | **2.5/1000 mg** | |
|---|---|---|
| **Components** | **mg/tab** | **%** |
| Metformin HCl | 1000.0 | 84.75 |

| Intragranular for Metformin | | |
|---|---|---|
| Mannitol 160C | 35.65 | 3.02 |
| Maize starch | 35.65 | 3.02 |
| Copovidone (Kollidon VA 64) | 94.4 | 8.00 |
| Linagliptin | 2.5 | 0.21 |
| p. Water* | q.s. | q.s. |
| *Pre-lubrication Weight* | *1168.2* | *99.00* |
| Magnesium stearate | 11.8 | 1.00 |
| *Core Tablet Weight* | **1180.0** | **100.0** |
| Opadry Pink | | 3.0 |
| ***Coated Tablet Weight*** | **1215.4** | **103.0** |

| | | |
|---|---|---|
| * Purified water is used in the granulation process (wet granulation) and it is not present in the tablet | | |

**Step 1:** The Metformin API, Mannitol, Maize Starch and some qty of Copovidone are sieved and mixed.
**Step 2:** Part of Copovidone and all the amount of Linagliptin are dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4 and mixing
**Step 6:** Lubrication: Sieving of Magnesium Stearate (#60mesh) and mixing
**Step 7:** Compression
**Step 8:** Coating

### Comparative Example 6

The composition of the tablet shown in the table below comprises polyvinylpyrrolidone (PVP) K30 as binder. The_tablet does not contain coated linagliptin granules. Linagliptin is added to the binding liquid which is used in the wet granulation.

| **Linagliptin/ Metformin** | **2.5/1000 mg** | |
|---|---|---|
| **Components** | **mg/tab** | **%** |
| Metformin HCl | 1000.0 | 84.75 |

| Intragranular for Metformin | | |
|---|---|---|
| Mannitol 160C | 35.65 | 3.02 |
| Maize starch | 35.65 | 3.02 |
| PVP K30 | 94.4 | 8.00 |
| Linagliptin | 2.5 | 0.21 |
| p. Water* | q.s. | q.s. |
| *Pre-lubrication Weight* | *1168.2* | *99.00* |
| Magnesium stearate | 11.8 | 1.00 |
| *Core Tablet Weight* | **1180.0** | **100.0** |
| Opadry Pink | | 3.0 |
| ***Coated Tablet Weight*** | **1215.4** | **103.0** |

| | | |
|---|---|---|
| * Purified water is used in the granulation process (wet granulation) and it is not present in the tablet | | |

**Step 1:** The Metformin API, Mannitol, Maize Starch and some qty of PVP K30 are sieved and mixed.
**Step 2:** Part of PVP K30 and all the amount of Linagliptin API are dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4 and mixing
**Step 6:** Lubrication: Sieving of Magnesium Stearate (#60mesh) and mixing
**Step 7:** Compression
**Step 8:** Coating

### Dissolution

The dissolution of both metformin hydrochloride and linagliptin from the tablet is tested at HCl 0.1N at 37° C ± 0.5°C using a USPII paddle apparatus with stirring speed of 50 rpm.

| | | | | | | |
|---|---|---|---|---|---|---|
| HCL 0.1N | Sample | RLD | Compar. Exam. 3 | Compar. Exam. 4 | Compar. Exam. 5 | Compar. Exam. 6 |
| | Dissolut. medium | HCL 0.1N | | | | |
| | Apparatus | PADDLES | | | | |
| | RPM | 50 | 50 | 50 | 50 | 50 |
| | Volume (mL) | 900 | 900 | 900 | 900 | 900 |
| Metformin | 10 min | 77.3 | 43.5 | 59.9 | 80.8 | 85.6 |
| | 15 min | 94.7 | 62.6 | 78.5 | 95.8 | 101.6 |
| | 30 min | 98.3 | 95.3 | 99.9 | 99.7 | 104.7 |
| | 45 min | 98.9 | 102.6 | 101.7 | 99.8 | 104.1 |
| Linagliptin | 10 min | 77.4 | 35.7 | 58.9 | 77.0 | 80.0 |
| | 15 min | 96.1 | 55.9 | 77.8 | 92.7 | 97.1 |
| | 30 min | 100.8 | 94.0 | 101.8 | 96.8 | 100.8 |
| | 45 min | 100.9 | 102.9 | 103.7 | 97.4 | 101.0 |

### Impurities

| | SAMPLE | RLD | RLD 4M 40°C/ 75% RH | Compar. Exam. 3 6M 40°C/ 75% RH | Compar. Exam. 4 1M 40°C/ 75% RH | Compar. Exam. 5 1M 40°C/ 75% RH | Compar. Exam. 6 4M 40°C/ 75% RH |
|---|---|---|---|---|---|---|---|
| Metformin | Maximum Unknown impurities (%) | 0.01 | 0.01 | 0.02 | 0.01 | 0.01 | 0.01 |
| | Maximum Known impurities (%) | 0.01 | 0.02 | 0.04 | 0.03 | 0.03 | 0.03 |
| | Total (%) | 0.02 | 0.10 | 0.12 | 0.04 | 0.04 | 0.04 |
| Linagliptin | Maximum Unknown impurities (%) | 0.14 | 0.16 | 0.21 | 1.68 | 1.15 | 0.99 |
| | Maximum Known impurities (%) | 0.34 | 0.23 | 26.16 | 0.14 | 0.31 | 0.23 |
| | Total (%) | 0.75 | 1.18 | 27.89 | 2.16 | 1.73 | 1.41 |
| Total (%) | | 0.77 | 1.28 | 28.01 | 2.20 | 1.77 | 1.45 |

| | | | | | | | |
|---|---|---|---|---|---|---|---|
| RH: Relative humidity | | | | | | | |

### Assay

| BATCH | Comparative Example 3 Zero Time | Comparative Example 3 1M/25 °C /60 RH | Comparative Example 3 1M/40°C /75 RH |
|---|---|---|---|
| %ASSAY (METFORMIN) | 99.3 | 97.7 | 98.2 |
| %ASSAY (LINAGLIPTIN) | 98.7 | 99.9 | 93.4 |

| BATCH | Comparative EXAMPLE 4 Zero time | Comparative EXAMPLE 4 1M/25 °C /60 RH | Comparative EXAMPLE 4 1M/40°C /75 RH |
|---|---|---|---|
| %ASSAY (METFORMIN) | 98.5 | 97.3 | 98.9 |
| %ASSAY (LINAGLIPTIN) | 101.1 | 99.5 | 94.4 |

| BATCH | Comparative EXAMPLE 5 ZERO TIME | Comparative EXAMPLE 5 1 M/25 °C /60 RH | Comparative EXAMPLE 5 1 M/40 °C /75 RH |
|---|---|---|---|
| %ASSAY (METFORMIN) | 100.9 | 99.3 | 97.8 |
| %ASSAY (LINAGLIPTIN) | 98.2 | 96.2 | 93.0 |

| BATCH | Comparative EXAMPLE 6 ZERO TIME | Comparative EXAMPLE 6 1 M/25 °C /60 RH | Comparative EXAMPLE 6 1 M/40 °C /75 RH |
|---|---|---|---|
| %ASSAY (METFORMIN) | 106.4 | 105.3 | 106.3 |
| %ASSAY (LINAGLIPTIN) | 103.1 | 101.8 | 97.0 |

| | | | |
|---|---|---|---|
| RH: Relative humidity | | | |

### Comparative Examples 7-9 with different Stabilizers

### Comparative EXAMPLE 7 with Opadry^{®} AMB as coating for linagliptin granules

Opadry^{®} AMB is a coating material that exhibits high performance moisture barrier film coating, is a fully formulated PVA-based immediate release system. It is based on Polyvinyl alcohol, titanium dioxide, talc, lecithin and xanthan gum.

| **Linagliptin/ Metformin** | **2.5/1000 mg** | |
|---|---|---|
| **Components** | **mg/tab** | **%** |
| Metformin HCl | 1000.0 | 81.17 |

| Intragranular for Metformin | | |
|---|---|---|
| MCC 101 | 100.0 | 8.12 |
| Hydroxypropyl cellulose | 37.5 | 3.04 |
| p. Water* | q.s. | q.s. |
| Linagliptin | 2.5 | 0.2 |
| Intragranular for linagliptin | | |
| MCC 102 | 37.5 | 3.04 |
| Crospovidone | 18.75 | 1.52 |
| Opadry^{®} AMB | 10.0 | 0.81 |
| Aerosil 200 | 12.0 | 0.97 |
| *Pre-lubrication Weight* | *1218.25* | *98.88* |
| Magnesium stearate | 13.75 | 1.12 |
| *Core Tablet Weight* | **1232.0** | **100.0** |
| Opadry^{®} Pink | | 3.0 |
| ***Coated Tablet Weight*** | **1269.0** | **103.0** |

| | | |
|---|---|---|
| MCC: Microcrystalline cellulose * Purified water is used in the granulation process (wet granulation) and it is not present in the tablet | | |

**Step 1:** The Metformin API and MCC 101 are sieved and mixed.
**Step 2:** Hydroxypropyl cellulose is dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4
**Step 6:** The Linagliptin and the extra granular excipients (MCC 102, Crospovidone, Opadry AMB and Aerosil) are sieved and mixed geometrically.
**Step 7:** The sized granules from step 5 are mixed with blend of step 6. (unlubricated ted blend)
**Step 8:** Lubrication: Sieving of Magnesium Stearate (#60mesh) and mixing
**Step 9:** Compression
**Step 10:** Coating

### Comparative EXAMPLE 8 with L-Histidine

The composition of the tablet shown in the table below comprises L-Histidine. The tablet does not contain coated linagliptin granules. Linagliptin is added to the binding liquid which is used in the wet granulation.

| **Linagliptin/ Metformin** | **2.5/1000 mg** | |
|---|---|---|
| **Components** | **mg/tab** | **%** |
| Metformin HCl | 1000.0 | 84.75 |

| Intragranular for Metformin | | |
|---|---|---|
| Mannitol 160C | 33.15 | 2.81 |
| Maize starch | 35.65 | 3.02 |
| PVP K30 | 94.4 | 8.00 |
| Linagliptin | 2.5 | 0.21 |
| L-Histidine | 2.5 | 0.21 |
| p. Water* | q.s. | q.s. |
| *Pre-lubrication Weight* | 1168.2 | *99.00* |
| Magnesium stearate | 11.8 | 1.00 |
| *Core Tablet Weight* | **1180.0** | **100.0** |
| Opadry^{®} Pink | | 3.0 |
| ***Coated Tablet Weight*** | **1215.4** | **103.0** |

| | | |
|---|---|---|
| PVP: Polyvinylpyrrolidone * Purified water is used in the granulation process (wet granulation) and it is not present in the tablet | | |

**Step 1:** The Metformin API, Mannitol, Maize Starch and PVP K30 are sieved and mixed.
**Step 2:** Linagliptin and L-Histidine are dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4 and mixing
**Step 6:** Lubrication: Sieving of Magnesium Stearate (#60mesh) and mixing
**Step 7:** Compression
**Step 8:** Coating

### Comparative EXAMPLE 9 with L-Glutamine

The composition of the tablet shown in the table below comprises L-Glutamine. The tablet does not contain coated linagliptin granules. Linagliptin is added to the binding liquid which is used in the wet granulation.

| **Linagliptin/ Metformin** | **2.5/1000 mg** | |
|---|---|---|
| **Components** | **mg/tab** | **%** |
| Metformin HCl | 1000.0 | 84.03 |

| Intragranular for Metformin | | |
|---|---|---|
| Mannitol 160C | 33.65 | 2.83 |
| Maize starch | 35.65 | 3.00 |
| PVP K30 | 94.4 | 7.93 |
| Linagliptin | 2.5 | 0.21 |
| L-Glutamine | 11.0 | 1.01 |
| p. Water | q.s. | q.s. |
| *Pre-lubrication Weight* | *1178.2* | *99.01* |
| Magnesium stearate | 11.8 | 0.99 |
| *Core Tablet Weight* | **1190.0** | **100.0** |
| Opadry Pink | | 3.0 |
| ***Coated Tablet Weight*** | **1225.7** | **103.0** |

| | | |
|---|---|---|
| PVP: Polyvinylpyrrolidone * Purified water is used in the granulation process (wet granulation) and it is not present in the tablet | | |

**Step 1:** The Metformin API, Mannitol, Maize Starch and some qty of PVP K30 & L-Glutamine are sieved and mixed.
**Step 2:** Part of PVP K30 & all the amount of L-Glutamine and Linagliptin API are dissolved in water
**Step 3:** Blend from step 1 is wet granulated with binding liquid from step 2
**Step 4:** Drying of the wet granules of step 3 (Target LOD: 1.8-2.5%)
**Step 5:** Sizing of the dried granules of step 4 and mixing
**Step 6:** Lubrication: Sieving of Magnesium Stearate (#60mesh) and mixing
**Step 7:** Compression
**Step 8:** Coating

### Dissolution (official)

| | Sample | RLD | Comparative Example 7 | Comparative Example 8 | Comparative Example 9 |
|---|---|---|---|---|---|
| HCL 0.1N | Diss. medium | HCL 0.1N | | | |
| | Apparatus | PADDLES | | | |
| | RPM | 50 | 50 | 50 | 50 |
| | Volume (mL) | 900 | 900 | 900 | 900 |
| Metformin | 10 MIN | 77.3 | 100.6 | 78.4 | 71.9 |
| | 15 MIN | 94.7 | 101.5 | 93.2 | 89.1 |
| | 30 MIN | 98.3 | 102.0 | 98.1 | 101.2 |
| | 45 MIN | 98.9 | 103.5 | 98.5 | 101.4 |
| Linagliptin | 10 MIN | 77.4 | 96.2 | 74.7 | 68.1 |
| | 15 MIN | 96.1 | 97.2 | 90.5 | 86.5 |
| | 30 MIN | 100.8 | 97.0 | 95.9 | 99.3 |
| | 45 MIN | 100.9 | 97.1 | 96.2 | 99.7 |

### Impurities

| | Sample | RLD | RLD | Compar. Example 7 | Compar. Example 8 | Compar, Example 9 |
|---|---|---|---|---|---|---|
| | | | 4M 40°C/ 75% RH | 15M 25°C/ 60% RH | 1M 40°C/ 75% RH | 1M 40°C/ 75% RH |
| Metformin | Maximum Unknown impurities (%) | 0.01 | 0.01 | 0.01 | 0.01 | 0.01 |
| | Maximum Known impurities (%) | 0.01 | 0.02 | 0.02 | 0.02 | 0.03 |
| | Total (%) | 0.02 | 0.10 | 0.05 | 0.07 | 0.05 |
| Linagliptin | Maximum Unknown impurities (%) | 0.14 | 0.16 | 0.06 | 0.21 | 0.04 |
| | Maximum Known impurities (%) | 0.34 | 0.23 | 1.14 | 0.20 | 0.32 |
| | Total (%) | 0.75 | 1.18 | 1.19 | 0.56 | 1.15 |
| Total (%) | | 0.77 | 1.28 | 1.24 | 0.63 | 1.20 |

### Assay

| BATCH | Comparative Example 7 | Comparative_Example 7 | |
|---|---|---|---|
| | 6M/25/60 | 6M/40/75 | |
| %ASSAY (METFORMIN) | 100.0 | 98.8 | |
| %ASSAY (LINAGLIPTIN) | 95.4 | 93.3 | |

| BATCH | Comparative Example 7 | Comparative Example 7 | Comparative Example 7 |
|---|---|---|---|
| | Zero time | 1M/25/60 | 1 M/40/75 |
| %ASSAY (METFORMIN) | 97.9 | 97.4 | 98.2 |
| %ASSAY (LINAGLIPTIN) | 94.2 | 92.7 | 91.0 |

| BATCH | Comparative Example 8 | Comparative EXAMPLE 8 | Comparative Example 8 |
|---|---|---|---|
| | Zero time | 1M/25/60 | 1M/40/75 |
| %ASSAY (METFORMIN) | 99.6 | 98.7 | 99.6 |
| %ASSAY (LINAGLIPTIN) | 100.9 | 95.5 | 92.4 |

### Example 10: MULTIMEDIA DISSOLUTION PROFILES OF TABLET OF EXAMPLE 2 AND OF JENTADUETO^{®} TABLETS (Reference listed Drug (RLD)

The tested tablets were subjected to dissolution tests at pH 1.2, pH 4.5 and pH 6.8; at 37° C using a USPII paddle apparatus with stirring speed of 50 rpm.

### HCl 0.1N = pH 1.2

| | SAMPLE | RLD | EXAMPLE 2 |
|---|---|---|---|
| HCL 0.1N | DISS. MED | HCL 0.1N | HCL 0.1N |
| | APPARATUS | PADDLES | PADDLES |
| | RPM | 50 | 50 |
| | VOLUME (mL) | 900 | 900 |
| METFORMIN | 10 MIN | 77.3 (3 | 92.8 |
| | 15 MIN | 94.7 | 96.0 |
| | 30 MIN | 98.3 | 97.6 |
| | 45 MIN | 98.9 | NA |
| LINAGLIPTIN | 10 MIN | 77.4 | 86.8 |
| | 15 MIN | 96.1 | 96.3 |
| | 30 MIN | 100.8 | 100.5 |
| | 45 MIN | 100.9 | NA |

### pH 4.5

| | SAMPLE | RLD | EXAMPLE 2 |
|---|---|---|---|
| pH 4.5 | DISS. MED | pH 4.5 | pH 4.5 |
| | APPARATUS | PADDLES | PADDLES |
| | RPM | 50 | 50 |
| | VOLUME (mL) | 900 | 900 |
| METFORMIN | 10 MIN | 73.8 | 92.5 |
| | 15 MIN | 93.8 | 96.2 |
| | 30 MIN | 102.4 | 98.0 |
| | 45 MIN | 102.7 | 98.4 |
| | 60 MIN | 102.8 | 98.9 |
| LINAGLIPTIN | 10 MIN | 70.9 | 79.9 |
| | 15 MIN | 91.8 | 91.8 |
| | 30 MIN | 101.0 | 98.2 |
| | 45 MIN | 101.2 | 100.2 |
| | 60 MIN | 101.4 | 101.3 |

### pH 6.8

| | SAMPLE | RLD | EXAMPLE 2 |
|---|---|---|---|
| pH 6.8 | DISS. MED | pH 6.8 | pH 6.8 |
| | APPARATUS | PADDLES | PADDLES |
| | RPM | 50 | 50 |
| | VOLUME (mL) | 900 | 900 |
| METFORMIN | 10 MIN | 77.5 | 92.9 |
| | 15 MIN | 95.4 | 95.9 |
| | 30 MIN | 101.7 | 98.4 |
| | 45 MIN | 101.8 | 99.1 |
| | 60 MIN | 102.0 | 99.1 |
| LINAGLIPTIN | 10 MIN | 74.8 | 68.9 |
| | 15 MIN | 93.2 | 85.3 |
| | 30 MIN | 100.0 | 95.9 |
| | 45 MIN | 100.2 | 98.3 |
| | 60 MIN | 100.4 | 99.6 |

### EXAMPLE 11: STABILITY DATA OF TABLET OF EXAMPLE 2

LIMITS
METFORMIN
Known impurities ≤ 0.15 %
highest individual unspecified impurity ≤ 0.10 %
LINAGLIPTIN
Known impurities ≤ 1.00 %
highest individual unspecified impurity ≤ 0.40 %

| | SAMPLE | RLD | RLD | EXAMPLE 2 | EXAMPLE 2 |
|---|---|---|---|---|---|
| | | | 4M 40°C/ 75% RH | ZERO TIME | 3M 40°C/ 75% RH |
| METFORMIN | Maximum Unknown impurities (%) | 0.01 | 0.01 | 0.01 | 0.01 |
| | Maximum Known impurities (%) | 0.01 | 0.02 | 0.01 | 0.02 |
| | Total (%) | 0.02 | 0.10 | 0.02 | 0.03 |
| LINAGLIPTIN | Maximum Unknown impurities (%) | 0.14 | 0.16 | 0.07 | 0.14 |
| | Maximum Known impurities (%) | 0.34 | 0.23 | <LOQ | 0.28 |
| | Total (%) | 0.75 | 1.18 | 0.14 | 0.54 |
| Total (%) | | 0.77 | 1.28 | 0.16 | 0.57 |

| | | | | | |
|---|---|---|---|---|---|
| LOQ: Limit of quantification | | | | | |

### Example 12: Dissolution profile of stability samples of tablet of example 2 after storage of 3months at 40°C/75% RH

| | | 3M 40°C /75% RH |
|---|---|---|
| HCL 0.1N =pH1.2 (1000/2.5) | SAMPLE | EXAMPLE 2 |
| | DISS. MED | HCL 0.1N |
| | APPARATUS | PADDLES |
| | RPM | 50 |
| | VOLUME (mL) | 900 |
| METFORMIN | 10 MIN | 94.5 |
| | 15 MIN | 96.8 |
| | 30 MIN | 98.3 |
| | 45 MIN | 98.1 |
| LINAGLIPTIN | 10 MIN | 86.8 |
| | 15 MIN | 96.7 |
| | 30 MIN | 102.2 |
| | 45 MIN | 102.7 |

The results of the above Examples clearly show that the present invention provides a tablet with excellent stability of both active ingredients. The stability of the active ingredients in a tablet according to present invention is better that the corresponding stability exhibited by tablets of the prior art. At the same time, the dissolution of both active ingredients from the tablet of the present invention is excellent in a wide pH range.

## Claims

1. A monolayer tablet comprising
(a) metformin granules comprising metformin hydrochloride and one or more excipients;
(b) linagliptin granules comprising linagliptin or a pharmaceutically acceptable salt thereof and one or more excipients, wherein the linagliptin granules are coated with a basic butylated methacrylate copolymer; and optionally,
(c) one or more extragranular excipients.

2. The monolayer tablet according to claim 1, wherein the tablet exhibits dissolution properties such that at least 90% of metformin and linagliptin are released from the tablet within 30 minutes; and wherein the dissolution test is performed at pH range from 1.2 to 6.8, at 37.0°C ± 0.5°C, using USP II paddle apparatus at stirring speed of 50 rpm.

3. The monolayer tablet according to claim 1 or 2, wherein the weight ratio of metformin hydrochloride to linagliptin, or to a pharmaceutically acceptable salt thereof, in the tablet ranges from 100:1 to 500:1.

4. The monolayer tablet according to any one of the preceding claims, wherein the metformin granules are prepared by wet granulation, or dry granulation.

5. The monolayer tablet according to any one of the preceding claims, wherein the linagliptin granules are prepared by wet granulation, or dry granulation.

6. The monolayer tablet according to any one of the preceding claims, wherein the metformin granules and the linagliptin granules are prepared by wet granulation.

7. The monolayer tablet according to any one of the preceding claims, wherein the D90 of metformin hydrochloride which is used in the preparation of the tablet is less than 400 µm, as measured by laser light diffraction as described in European Pharmacopoeia, 11^{th} Edition, 2.9.31.

8. The monolayer tablet according to any one of the preceding claims, wherein the D90 of linagliptin, or a pharmaceutically acceptable salt thereof, which is used in the preparation of the tablet is less than 200 µm, preferably less than 50 µm, as measured by laser light diffraction as described in European Pharmacopoeia, 11^{th} Edition, 2.9.31.

9. The monolayer tablet according to any one of the preceding claims, wherein the metformin granules comprise metformin hydrochloride, a filler and a binder.

10. The monolayer tablet according to any one of the preceding claims, wherein the metformin granules comprise metformin hydrochloride, microcrystalline cellulose and hydroxypropyl cellulose.

11. The monolayer tablet according to any one of the preceding claims, wherein the linagliptin granules comprise linagliptin, or a pharmaceutically acceptable salt thereof, a filler, a disintegrant and a binder.

12. The monolayer tablet according to any one of the preceding claims, wherein the linagliptin granules comprise linagliptin, maize starch, mannitol, pregelatinized starch and povidone.

13. The monolayer tablet according to any one of the preceding claims, wherein the ratio of 2-(dimethylamino)ethyl methacrylate groups to butyl methacrylate and methyl methacrylate groups in the basic butylated methacrylate copolymer is about 2:1:1.

14. The monolayer tablet according to any one of the preceding claims, wherein the composition is free of a basic amino acid, a stabilizer of metformin, a stabilizer of linagliptin, and copovidone.

15. A process for the preparation of a monolayer tablet according to any one of claims 1 to 14, wherein the process comprises
(a) granulating metformin hydrochloride and one or more excipients to prepare metformin granules;
(b) granulating linagliptin or a pharmaceutically acceptable salt thereof and one or more excipients to prepare linagliptin granules and coating the linagliptin granules with basic butylated methacrylate copolymer;
(c) mixing metformin granules prepared in (a) and linagliptin granules prepared in (b);
(d) optionally adding one or more extragranular excipients; and
(f) compressing into tablets.
